# FASCICULE DE BREVET EUROPEEN

(11) **EP 2 150 623 B1**
(45) Date de publication et mention de la délivrance du brevet: **31.10.2012**
(21) Numéro de dépôt: 08805655.1
(22) Date de dépôt: 06.06.2008
(51) Int. Cl.: C12Q 1/56, G01N 33/86

(54) **MÉTHODE DE MESURE DE L'ACTIVITÉ PLASMINE DES MICROPARTICULES PRÉSENTES DANS UN ÉCHANTILLON DE FLUIDE BIOLOGIQUE ET UTILISATION**
VERFAHREN ZUR MESSUNG DER PLASMINAKTIVITÄT VON IN EINER PROBE EINER BIOLOGISCHEN FLÜSSIGKEIT VORHANDENEN MIKROPARTIKELN UND ANWENDUNG DAVON
METHOD FOR MEASURING THE PLASMINE ACTIVITY OF MICROPARTICLES PRESENT IN A SAMPLE OF A BIOLOGICAL FLUID AND USE THEREOF

(30) Priorité: 07.06.2007 FR 0704060
(43) Date de publication de la demande: 10.02.2010
(73) Titulaire: Institut National de la Santé et de la Recherche Médicale (INSERM), 75013 Paris Cedex 13 (FR); Assistance Publique Hôpitaux de Marseille, 13354 Marseille Cedex 5 (FR); Université de la Méditerranée, 13007 Marseille (FR); Universite De Caen Basse-Normandie, 14032 Caen Cedex (FR)
(72) Inventeur: ANGLES CANO, Eduardo, F-75015 Paris (FR); LACROIX, Romaric, F-13010 Marseille (FR); MALATERRE, Florence, F-13001 Marseille (FR); DIGNAT-GEORGE, Françoise, F-13008 Marseille (FR)
(74) Mandataire: Galup, Cédric Olivier Nicolas
(86) Numéro de dépôt international: PCT/FR2008/000767
(87) Numéro de publication internationale: WO 2009/004189

(56) Documents cités:
- WO-A-2006/062945
- WO-A-2006/072602
- FR-A- 2 795 820
- FR-A- 2 827 872
- US-A- 5 776 452
- US-A1- 2005 282 228
- GRAVES LAURA E ET AL: "Proinvasive properties of ovarian cancer ascites-derived membrane vesicles" CANCER RESEARCH, vol. 64, no. 19, 1 octobre 2004 (2004-10-01), pages 7045-7049, XP002461201 ISSN: 0008-5472
- FREYSSINET: "CELLULAR MICROPARTICLES: WHAT ARE THEY BAD OR GOOD FOR" JOURNAL OF THROMBOSIS AND HAEMOSTASIS, BLACKWELL PUBLISHING, OXFORD, GB, vol. 1, 2003, pages 1655-1662, XP008070838 ISSN: 1538-7933
- CHAZOV E I ET AL: "Experimental study of biosoluble drugs. Thrombus lysis with biosoluble immobilized fibrinolysin in experiment" THROMBOSIS RESEARCH, TARRYTOWN, NY, US, vol. 12, no. 5, 1 mai 1978 (1978-05-01), pages 809-816, XP022881035 ISSN: 0049-3848 [extrait le 1978-05-01]
- SIMAK J ET AL: "Circulating endothelial microparticles in acute ischemic stroke: A link to severity, lesion volume and outcome" JOURNAL OF THROMBOSIS AND HAEMOSTASIS 2006 UNITED KINGDOM, vol. 4, no. 6, 2006, pages 1296-1302, XP002461202 ISSN: 1538-7933 1538-7836
- LAMANUZZI LEILA B ET AL: "Neutrophils stimulated by apolipoprotein(a) generate fragments that are stronger inhibitors of plasmin formation than apo(a)." THROMBOSIS AND HAEMOSTASIS NOV 2004, vol. 92, no. 5, novembre 2004 (2004-11), pages 1066-1075, XP002461203 ISSN: 0340-6245 cité dans la demande
- DIGNAT-GEORGE, F. ET AL: "Numeration of circulating microparticles of various cellular origin by flow cytometry" JOURNAL OF THROMBOSIS AND HAEMOSTASIS, vol. 2, 2004, pages 1844-1845, XP002461204
- HUGEL ET AL.: "Solid-phase capture assay for the determination of cellular microparticles and associated deleterious potentials" JOURNAL OF THROMBOSIS AND HAEMOSTASIS, vol. 2, 2004, pages 1846-1847, XP002461205
- COMBES V ET AL: "IN VITRO GENERATION OF ENDOTHELIAL MICROPARTICLES AND POSSIBLE PROTHROMBOTIC ACTIVITY IN PATIENTS WITH LUPUS ANTICOAGULANT" JOURNAL OF CLINICAL INVESTIGATION, NEW YORK, NY, US, vol. 104, no. 1, juillet 1999 (1999-07), pages 93-102, XP000913435 ISSN: 0021-9738
- DOLO V ET AL: "Matrix-degrading proteinases are shed in membrane vesicles by ovarian cancer cells in vivo and in vitro." CLINICAL & EXPERIMENTAL METASTASIS MAR 1999, vol. 17, no. 2, mars 1999 (1999-03), pages 131-140, XP002461206 ISSN: 0262-0898
- MOREL ET AL: "Les microparticules circulantes : roles physiologiques et implications dans les maladies inflammatoires et thrombotiques" REVUE DE MEDECINE INTERNE, CMR, ASNIERES, FR, vol. 26, no. 10, 1 octobre 2005 (2005-10-01), pages 791-801, XP005117584 ISSN: 0248-8663
- NOVOKHATNY V V ET AL: "Locally delivered plasmin: why should it be superior to plasminogen activators for direct thrombolysis?" TRENDS IN PHARMACOLOGICAL SCIENCES, ELSEVIER, HAYWARTH, GB, vol. 25, no. 2, 1 février 2004 (2004-02-01), pages 72-75, XP004489197 ISSN: 0165-6147
- GINESTRA A ET AL: "Urokinase plasminogen activator and gelatinases are associated with membrane vesicles shed by human HT1080 fibrosarcoma cells." THE JOURNAL OF BIOLOGICAL CHEMISTRY 4 JUL 1997, vol. 272, no. 27, 4 juillet 1997 (1997-07-04), pages 17216-17222, XP002511444 ISSN: 0021-9258
- A. S. Shet ET AL: "Use of flow cytometry to enumerate and assign origin of circulating cell-derived microparticles", Journal of Thrombosis and Haemostasis, vol. 2, no. 10, 1 October 2004 (2004-10-01), pages 1848-1850, XP55019741, ISSN: 1538-7933, DOI: 10.1111/j.1538-7836.2004.00941.x
- E. Biro ET AL: "A flow cytometric method of microparticle analysis", Journal of Thrombosis and Haemostasis, vol. 2, no. 10, 1 October 2004 (2004-10-01), pages 1843-1844, XP55019740, ISSN: 1538-7933, DOI: 10.1111/j.1538-7836.2004.00937.x
- JY W ET AL: "Measuring circulating cell-derived microparticles", JOURNAL OF THROMBOSIS AND HAEMOSTASIS, BLACKWELL PUBLISHING, OXFORD, GB, vol. 2, no. 10, 1 October 2004 (2004-10-01), pages 1842-1843, XP002501566, ISSN: 1538-7933, DOI: 10.1111/J.1538-7836.2004.00936.X

## Description

L'invention a pour objet une méthode de mesure de l'activité plasmine des microparticules, particulièrement des microparticules circulantes, présentes dans un échantillon de sang, telle que définie dans la revendication 1, , ladite méthode pouvant servir comme méthode de diagnostic ou comme méthode de suivi d'un traitement.

Les microparticules résultant de bourgeonnements de la membrane cellulaire ont été décrites dans divers modèles cellulaires et dans de nombreuses conditions pathologiques en tant que marqueurs fiables de l'activation et/ou de l'apoptose cellulaire.

Particulièrement, les inventeurs ont initialement décrit le relargage de ces microparticules par les cellules endothéliales en réponse à une stimulation inflammatoire et l'augmentation de la quantité de microparticules endothéliales circulantes chez des patients présentant un risque de thromboses. Depuis, des niveaux élevés de microparticules endothéliales circulantes ont été décrits dans différentes conditions pathologiques telles que les syndromes coronariens, l'insuffisance rénale, le diabète, le syndrome des antiphospholipides (SAPL), le purpura thrombotique thrombocytopénique (PTT) ou encore la drépanocytose, désordres dans lesquels la présence des microparticules reflète des disfonctionnements endothéliaux et est indicative d'un mauvais pronostic.

Les microparticules exprimant divers composants bioactifs provenant des cellules dont elles sont issues, elles peuvent présenter un large éventail d'activités biologiques capables de moduler des fonctions de cellules endothéliales ou sanguines, d'influencer l'homéostasie vasculaire et de participer aux réponses inflammatoires ou à l'angiogénèse.

Par exemple, les microparticules, particulièrement les microparticules circulantes, présentent des surfaces phospholipidiques procoagulantes impliquées dans l'assemblage et l'activation des facteurs de coagulation.

De même, la participation des microparticules, particulièrement les microparticules circulantes, dans la génération de thrombine résulte de leur capacité à exprimer, transférer ou induire le facteur tissulaire dans le compartiment vasculaire.

Parmi les principaux régulateurs de l'équilibre hémostatique, le système d'activation du plasminogène est la principale voie physiologique de la dissolution du caillot de fibrine. Cette voie facilite également l'angiogénèse en aidant à la protéolyse des composants de la matrice extracellulaire.

La conversion du plasminogène en plasmine active dépend de deux sérines - protéases : l'activateur tissulaire du plasminogène (t-PA : tissue-type plasminogen activator) qui dans les vaisseaux est principalement impliqué dans la fibrinolyse et l' urokinase (u-PA ; urokinase-type plasminogen activator) qui liée à son récepteur spécifique, uPAR, est impliquée dans la protéolyse péricellulaire.

La génération de plasmine induite par l'uPA et l'activation des métalloprotéinases de la matrice (MMP) qui en résulte, favorise la migration de cellules au travers de la matrice interstitielle et participe aux processus tels que le remodelage tissulaire, l'invasion métastatique et l'angiogénèse.

Une activation non contrôlée et/ou excessive du plasminogène peut avoir des conséquences délétères en induisant le détachement cellulaire et/ou l'apoptose cellulaire. On comprend donc que la régulation de l'expression de la plasmine à la surface des cellules, particulièrement des cellules endothéliales est d'importance critique dans la régulation de l'homéostasie vasculaire.

Le document Graves Laura E et al, (Cancer Research 64, 7045-7049, 1 octobre 2004) décrit une méthode de mesure de l'activité plasmine des vésicules liées aux membranes isolées d'échantillons d'ascites. Le document de JY W. et al. (« Measuring circulating cell-derived microparticles », Journal of thrombosis and haemostasis, vol. 2, no. 10, 1 octobre 2004, pages 1842-1843) décrit quant à lui différentes méthodes pour isoler les microparticules.

A la lecture de ce qui précède on comprend donc l'intérêt qu'il y a d'une part à pouvoir évaluer la génération de l"activité plasmine" des microparticules, particulièrement les microparticules circulantes, dans un fluide biologique, particulièrement dans un fluide biologique en situation d'écoulement, très particulièrement le sang, ou dans des extraits tissulaires et d'autre part à pouvoir moduler cette activité.

Par "fluide biologique" on entend tout liquide corporel extractible dont par exemple le sang, le liquide céphalorachidien (LCR), le liquide broncho-alvéolaire (LBA), l'urine, le liquide synovial, le lait maternel, la salive, les larmes, le liquide séminal, les liquides d'ascite, l'épanchement pleural, le liquide amniotique.

Par "fluide biologique en situation d'écoulement" on entend tout liquide corporel qui s'écoule dans ou hors du corps naturellement dont par exemple le sang circulant, le lait maternel, l'urine, la salive, les larmes, le liquide séminal, l'écoulement menstruel et tout autre écoulement séreux et/ou muqueux

Parmi les sources d'extrait tissulaire on peut citer les plaques d'athérome ou tout autre tissue obtenu par chirurgie.

Par "activité plasmine " on doit comprendre dans le présent texte la capacité d'un échantillon fluide biologique, particulièrement un fluide biologique en situation d'écoulement, contenant des microparticules, particulièrement les microparticules circulantes, à générer de la plasmine, quel que soit le mécanisme mis en oeuvre.

A titre de méthode de diagnostic, mesurée dans l'échantillon fluide biologique contenant les microparticules, particulièrement les microparticules circulantes, et comparée à la mesure de cette même capacité dans un échantillon témoin obtenu d'individus dits normaux, c'est-à-dire ne présentant aucune pathologie, la valeur de l"'activité plasmine de l'échantillon testé", si elle est significativement supérieure à celle du témoin reflétera par exemple et sans limitation, pour un individu, un risque plus ou moins élevé à subir par exemple des accidents vasculaires causés par une instabilité accrue des plaques d'athérome, un risque plus ou moins élevé pour un individu atteint d'un cancer à subir une invasion métastasique ou encore pour un individu un risque plus ou moins élevé à subir un accident vasculaire cérébral et ses conséquences délétères sur le fonctionnement cérébral. Si cette valeur de l"'activité plasmine de l'échantillon testé" est inférieure à celle du témoin, alors elle reflétera par exemple pour l'individu dont le sang a été testé un risque accru de thrombose.

A titre de suivi d'un traitement, mesurée dans les microparticules, particulièrement les microparticules circulantes, d'un 'échantillon fluide biologique, particulièrement un fluide biologique en situation d'écoulement, d'un individu en cours de traitement, et comparée à la mesure de cette même capacité dans un échantillon témoin obtenu du même individu avant le traitement ou précédemment dans le traitement, la valeur de l"'activité plasmine de l'échantillon testé" permet de suivre l'évolution de la réponse dudit individu en fonction du traitement qui lui est administré.

Cependant, Il existe toujours un besoin en test simple, efficace et fiable, des risques encourus par un patient, liés à une trop forte ou à une trop faible activité plasmine de son sang ou encore un test simple du suivi de l'évolution d'un traitement visant à moduler l'activité plasmine des microparticules, particulièrement les microparticules circulantes, d'un fluide biologique, particulièrement un fluide biologique en situation d'écoulement, d'un individu.

C'est un des buts de la présente invention que de fournir un tel test.

En effet, après de longs travaux et de manière surprenante, les inventeurs ont montré et ce à leur connaissance pour la première fois, que les microparticules circulantes présentes dans un fluide biologique, particulièrement un fluide biologique en situation d'écoulement, particulièrement dans le sang, d'un individu, sont porteuses d'une activité biologique leur conférant la capacité à générer de la plasmine.

Sur la base de cette découverte, la présente description a pour objet une méthode de mesure de l'activité plasmine des microparticules, particulièrement les microparticules circulantes, d'un échantillon de fluide biologique, particulièrement un fluide biologique en situation d'écoulement, particulièrement de sang, préalablement prélevé, dans lequel
- dans une première étape on isole les microparticules, particulièrement les microparticules circulantes, présentes dans ledit échantillon,
- dans une deuxième étape on mesure, par tout moyen approprié, la capacité desdites microparticules isolées à l'étape 1 à générer de la plasmine et
- dans une troisième étape on compare le résultat de la mesure obtenu à l'étape 2 au résultat d'une mesure identique réalisée dans les mêmes conditions sur un échantillon de fluide biologique identique témoin.

Ainsi très précisément l'objet de l'invention est tel que défini par la revendication 1.

Selon une variante de l'invention, le fluide biologique identique témoin peut être un fluide biologique identique à celui testé mais provenant d'au moins un individu considéré comme sain, c'est-à-dire ne présentant pas de pathologie, à tout le moins pas la pathologie dont souffre l'individu dont le fluide biologique est testé, et permettant alors d'évaluer les risques dudit individu au regard d'une valeur considérée comme normale.

Selon une autre variante de l'invention, le fluide biologique identique témoin peut être le même fluide biologique que celui testé, provenant du même individu mais obtenu dans un prélèvement antérieur à celui ayant donné l'échantillon testé, par exemple avant le début d'un traitement, afin de pouvoir établir un suivi de l'évolution de la capacité des microparticules à générer de la plasmine par exemple au cours d'un traitement.

Selon l'invention, la première étape de la méthode (isolement des microparticules, particulièrement les microparticules, circulantes présentes dans l'échantillon), peut être réalisée selon tout procédé compatible avec l'isolement de telles microparticules. Par exemple on citera la centrifugation à hautes vitesses ou encore des techniques de biocapture quel que soit le support capteur (par exemple des anticorps, par exemple de l'annexine V).

Selon la première étape de la méthode selon l'invention, les microparticules, particulièrement les microparticules circulantes, peuvent être isolées par une succession de centrifugations et d'ultracentrifugations dans un procédé selon lequel dans
- une étape 1A on centrifuge un volume compris entre 500 µl et 5 ml, préférentiellement entre 1 ml et 2 ml, d'un échantillon de fluide biologique, particulièrement un fluide biologique en situation d'écoulement, par exemple de sang, préalablement prélevé, à une vitesse comprise entre 1000 g et 2000 g, préférentiellement entre 1200 g et 1800g, pendant un temps compris entre 5 minutes et 20 minutes, préférentiellement entre 10 et 15 minutes, à une température comprise entre 2 et 6°C, préférentiellement entre 3 et 5°C ;
- dans une étape 1B, on centrifuge le surnageant obtenu à l'étape 1A à une vitesse comprise entre 10000 g et 20000 g, préférentiellement entre 12000 g et 15000g, pendant un temps compris entre 1 minute et 5 minutes, préférentiellement entre 2 et 3 minutes, à une température comprise entre 2 et 6°C, préférentiellement entre 3 et 5°C ;
- dans une étape 1C on centrifuge le surnageant obtenu à l'étape 1 B à une vitesse comprise entre 15000 g et 25000 g, préférentiellement entre 18000 g et 22000g, pendant un temps compris entre 45 et 120 minutes, préférentiellement entre 60 et 100 minutes, à une température comprise entre 2 et 6°C, préférentiellement entre 3 et 5°C ;
- dans une étape 1 D on reprend le culot obtenu à l'étape 1C dans un volume compris entre 250 µl et 4 ml, préférentiellement entre 1 et 2 ml, de tampon phosphate salin (PBS) et on centrifuge le mélange à une vitesse comprise entre 15000 g et 25000 g, préférentiellement entre 18000 g et 22000g, pendant un temps compris entre 45 et 120 minutes, préférentiellement entre 60 et 100 minutes, à une température comprise entre 2 et 6°C, préférentiellement entre 3 et 5°C ;
- dans une étape 1E on reprend le culot obtenu à l'étape 1 D dans un volume compris entre 250 µl et 4 ml, préférentiellement entre 1 et 2 ml, de tampon phosphate salin (PBS) et on centrifuge le mélange à une vitesse comprise entre 15000 g et 25000 g, préférentiellement entre 18000 g et 22000g, pendant un temps compris entre 45 et 120 minutes, préférentiellement entre 60 et 100 minutes, à une température comprise entre 2 et 6°C, préférentiellement entre 3 et 5°C ;
- dans une étape 1 F on reprend le culot obtenu à l'étape 1 E dans un volume compris entre 20 µl et 500 µl préférentiellement entre 50 et 100 µl, de tampon phosphate salin (PBS).

La suspension de microparticules, particulièrement les microparticules circulantes, obtenue à l'étape 1F peut être utilisée immédiatement pour analyse ou peut être conservée préférentiellement à -80°C.

Selon l'invention, la deuxième étape de la méthode (mesure de la capacité des microparticules, particulièrement les microparticules circulantes, à générer de la plasmine) peut être réalisée directement sur la quantité de microparticules obtenues à l'issu de la première étape. Préférentiellement selon l'invention, la deuxième étape de la méthode peut être réalisée sur une quantité déterminée de microparticules obtenues à l'issu de la première étape, ladite quantité pouvant être comprise entre 10000 et 1000000 microparticules, préférentiellement entre 100000 et 300000 microparticules. Dans ce cas la méthode selon l'invention peut comporter une étape supplémentaire (étape 1 bis) de dénombrement des microparticules obtenues à l'issu de la première étape, ladite étape de dénombrement intervenant entre la première et la deuxième étape de la méthode selon l'invention.

Ladite étape de dénombrement peut être réalisée selon l'invention selon tout procédé connu de dénombrement de microparticules. Avantageusement, le dénombrement des microparticules peut être réalisé par cytométrie de flux selon les protocoles classiquement utilisés dans l'art antérieur, par exemple ceux décrits dans la demande de brevet français FR-A-2795820, ou encore par un test de détection basé sur l'activité procoagulante des microparticules (Zymuphen MP-activity, Hyphen BioMed) ou encore un dosage protéique. Préférentiellement selon l'invention, on dénombre les microparticules par cytométrie de flux.

Selon l'invention, la deuxième étape de la méthode, c'est-à-dire la mesure de la capacité desdites microparticules, particulièrement les microparticules circulantes, isolées à l'étape 1 à générer de la plasmine peut être déterminée soit par la mesure de la quantité de plasmine spontanément présente sur les microparticules, soit par mesure de la quantité de plasmine susceptible d'être produite par ces microparticules.

Lesdites mesures de la plasmine peuvent être réalisée par toute méthode connue.

Selon une variante de l'étape 2 de la méthode selon l'invention, la mesure de la quantité de plasmine spontanément présente sur les microparticules, particulièrement les microparticules circulantes, peut être réalisée par toute méthode connue, comme par exemple une mesure immunologique (FASEB J 2003, 17 : 1301-3) (ELISA ou Western blot) à l'aide d'anticorps antiplasmin(ogèn)e (par exemple TC12040, Technoclone, Austria ou product 3641, American Diagnostica) ou encore par spectrophotométrie, par lecture de l'absorbance de l'échantillon à 405 nm à l'aide de substrats chromogènes sélectifs de la plasmine (par exemple CBS0065, Stago).

Selon une autre variante de l'étape 2 de la méthode selon l'invention, la mesure de la quantité de plasmine susceptible d'être produite par les microparticules, particulièrement les microparticules circulantes, peut être réalisée selon le procédé décrit dans Thromb Haemost 2004 ; 92 :1066-75, dans lequel
- dans une étape 2-1, on ajoute aux microparticules obtenues à l'étape 1 ou à l'étape 1 bis de la méthode selon l'invention, du plasminogène, avantageusement purifié, en une quantité finale comprise entre 0, 1 µM et 2, 0 µM, préférentiellement entre 0, 5 µM et 1 µM, et d'un substrat chromogénique sélectif de la plasmine, comme par exemple le (méthyl-malonyl)-hydroxypropylarginine-p-nitroanilide (CBS0065) commercialisé par la société STAGO (France), en une quantité finale comprise entre 0, 50 mM et 1, 0 mM, préférentiellement entre 0, 65 mM et 0, 85 mM ;
- dans une étape 2-2 on incube, le mélange obtenu à l'étape 2-1, par exemple dans une étuve sèche, à une température comprise entre 25°C et 45°C, préférentiellement 30°C et 40°C, pendant un temps compris entre 30 minutes et 90 minutes, préférentiellement entre 50 et 70 minutes et
- dans une étape 2-3 on mesure la quantité de plasmine susceptible d'être produite est détectée par photométrie par lecture de l'absorbance de l'échantillon à 405 nM (par exemple dans un lecteur de plaques de 96 puits comme le lecteur de microplaque MR 700 Dynex).

Selon une variante de l'étape 2-1, le substrat sélectif de la plasmine peut être un substrat fluorescent comme par exemple le H-D-Val-Leu-Lys-7-amido-4-methylcoumarin (Bachem, Bubendorf, Switzerland) ou le D-AFK-ANSNH-C4H9.2HB (Haematologic Technologies Inc, Vermont USA).

Selon une variante de l'étape 2-2 le mélange obtenu à l'étape 2-1 est déposé dans un lecteur de plaques, thermostaté à 37°C, qui mesure la cinétique de formation de plasmine par mesure de l'absorbance à 405 nm en fonction du temps.

Selon l'invention, la deuxième étape de la méthode peut être réalisée sur tout support compatible avec les incubations et les mesures à réaliser. A cet égard on peut citer des cupules à fonds ronds ou des cupules à fonds plats, par exemple les cupules de plaques 48 ou 96 puits en polystyrène ou en chlorure de polyvinyle. Préférentiellement l'étape 2 de la méthode selon l'invention est réalisée dans de cupules à fond rond ou des cupules à fond plat de plaques à 96 puits.

Selon l'invention, la mesure de la quantité de plasmine susceptible d'être produite par les microparticules, particulièrement les microparticules circulantes, ou de la quantité de plasmine spontanément présente sur les microparticules, particulièrement les microparticules circulantes, peut être réalisée dans un volume final compris entre 25 µl et 150 µl, préférentiellement entre 50 µl et 100 µl, ajusté par exemple à l'aide de tampon phosphate salin (PBS) additionné d'albumine de sérum bovin (Bovin Serum Albumin: BSA) à une concentration comprise entre 1, 0 et 3, 0 mg/ml, préférentiellement entre 1, 5 et 2, 5 mg/ml. Le nombre de microparticules à tester par puits peut être compris entre 50 000 et 400 000 particules, préférentiellement 100 000 à 200 000 particules par puits. Avantageusement, lorsque la mesure est réalisée dans des cupules à fond rond, le volume final est préférentiellement de 50 µl et quand la mesure est réalisée dans des cupules à fond plats, le volume final est préférentiellement de 100 µl. Selon l'invention, les résultats sont exprimés en quantité de plasmine produite par nombre de microparticules.

Dans un échantillon de fluide biologique, particulièrement un fluide biologique en situation d'écoulement, très particulièrement de sang, les microparticules, particulièrement les microparticules circulantes, représentent une population globale des microparticules dont on a vu précédemment qu'elles ont pour origine un bourgeonnement cellulaire et qu'elles peuvent être issues de nombreux types cellulaires différents. A cet égard on peut citer des microparticules issues de cellule endothéliales, de cellules hématopoïétiques.

Ainsi, selon le type cellulaire dont elles sont issues, les microparticules, particulièrement les microparticules circulantes, seront porteuses de caractéristiques propres au type cellulaire dont elles sont issues. Sur cette base il est possible d'isoler les microparticules en fonction de leur origine et ainsi de générer des populations distinctes de microparticules d'un seul type. Or, il peut être intéressant de ne réaliser la mesure de l'activité plasmine que sur un type particulier de microparticules.

Ainsi, selon un mode de réalisation particulier de l'invention, la méthode peut comprendre en outre une étape 1ter d'isolement des microparticules en fonction de leur origine. Cette étape peut être réalisée après la première étape de la méthode selon l'invention, c'est-à-dire après l'étape 1 ou l'étape 1 bis de la dite méthode, préférentiellement après l'étape 1 et avant l'étape 1 bis.

L'isolement des microparticules d'intérêt peut être réalisé par toute méthode connue de l'art antérieur. On citera à cet égard une procédure de tri cellulaire par cytométrie ou encore l'immunoséparation magnétique. Préférentiellement selon l'invention on utilise la méthode d'immunoséparation magnétique.

Selon encore une autre variante de la méthode selon l'invention, il est possible d'immobiliser les microparticules isolées à l'étape 1 au support sur lequel l'étape 2 va être réalisée. L'immobilisation des microparticules peut être réalisée selon tout procédé connu de l'art antérieur, particulièrement celui décrit dans la demande internationale WO-A-96/03655. Par exemple il est possible de préparer le support utilisé à l'étape 2 de la méthode selon l'invention en couvrant sa surface à l'aide d'un composé apte à immobiliser les microparticules puisque reconnaissant un élément de la surface desdites microparticules. On peut citer à cet égard l'annexine V qui reconnaît les phospholipides procoagulants, ou encore les anticorps spécifiques des complexes glycoprotéiques conformationnels actifs et/ou fonctionnels des membranes GPIIb/GPIIIa, ou encore les récepteurs adhésifs des monocytes ou des lymphocytes LFA-1 ou encore la thrombomoduline endothéliale ou encore le CD146. Selon une autre variante de la méthode selon l'intention il est possible d'immobiliser les microparticules à l'aide d'un polycation comme la poly-L-lysine.

L'invention a également pour objet l'utilisation de microparticules circulantes, présentes dans un échantillon de sang, dans une méthode de mesure de l'activité plasmine dudit échantillon de sang selon l'une des revendications 1 à 18.

L'invention a encore pour objet l'utilisation d'une méthode selon l'une des revendications 1 à 18 dans une méthode de diagnostic, chez un l'individu dont le sang est issu,
- du risque plus ou moins élevé à subir des accidents vasculaires causés par exemple par une instabilité accrue des plaques d'athérome, ou encore
- du risque plus ou moins élevé pour ledit individu atteint d'un cancer à subir une invasion métastasique, ou encore
- du risque plus ou moins élevé pour ledit individu à subir un accident vasculaire cérébral, ses complications hémorragiques.
- du risque pour ledit individu de subir une thrombose.

Les inventeurs ont par ailleurs pu montrer que les microparticules circulantes, particulièrement les microparticules issues de cellules endothéliales, contenues dans un fluide biologique, particulièrement un fluide biologique en situation d'écoulement, par exemple du sang, porteuses d'une activité plasmine au sens de l'invention, présentent une grande résistance à l'inactivation, particulièrement à la neutralisation ou l'inhibition par les inhibiteurs d'enzymes protéolytiques présentes dans le fluide biologique. Cette propriété confère auxdites microparticules circulantes la capacité à véhiculer l'activité plasmine au travers de l'organisme par le fluide biologique jusqu'au lieu où la présence de la plasmine développe son activité, sans risque d'inhibition, à tout le moins avec un risque d'inhibition extrêmement diminué. A cet égard on sait que la plasmine native circulante est rapidement inhibée dans les fluides biologiques. Lesdites microparticules peuvent alors être assimilées à un vecteur de l'activité plasmine, ce qui permet d'envisager leur utilisation en tant que tel, une fois purifiées ou semi-purifiées. De la même façon que les microparticules portant le facteur tissulaire sont potentiellement utiles dans le traitement de maladies hémorragiques congénitales comme l'hémophilie (Nature Medicine 2003, 9 : 1020-1025).

Par purifiée ou semi-purifiées, on entend que les microparticules sont utilisées après avoir subi au moins une étape de purification.

Ainsi la description a pour objet l'utilisation de microparticules, particulièrement les microparticules circulantes, issues de cellules endothéliales, purifiée ou semi-purifiées, à titre de vecteur de l'activité plasmine.

La description a encore pour objet l'utilisation de microparticules, particulièrement les microparticules circulantes, particulièrement des microparticules issues de cellules endothéliales, purifiée ou semi-purifiées, à titre de médicament, particulièrement de médicament à activité protéolytique ou antithrombotique.

D'autres caractéristiques et avantages de l'invention apparaîtront à la lecture des exemples suivants qui ne sont donnés qu'à titre illustratif et ne limitent en rien la présente invention.

### Exemples

### Exemple 1 : Mise en évidence de l'activité plasmine portée par les microparticules de cellules endothéliales en culture :

### 1-A : Préparation de microparticules de cellules endothéliales :

Des cellules de lignée endothéliale microvasculaire humaine HMEC-1 (J. Invest. Dermatol. 1992 ; 99 : 683-90) ont été cultives à sous confluence en milieu MCDB 131 (Invitrogen Life Technologies, Cergy Pontoise, France) additionné de 10% de sérum de veau foetal exempt de microparticule (FCS), 10 ng/ml d'EGF humain recombinant (Upstate Cell Signaling Solutions, Lake Placid, NY, USA) et de 1 µg/ml d'hydrocortisone (Sigma, St Quentin Fallavier, France).

Les microparticules endothéliales (EMP) ont été purifiées à partir du milieu de culture des cellules HMEC-1 stimulées pendant 48 h avec 100 ng/ml de TNF-α (PeproTech Inc, Rocky Hill, NJ, USA) selon les conditions décrites dans J. Clin. Invest. 1999 Jul; 104(1):93-102)

Les surnageants de culture ont été centrifugés 4300g pendant 5 minutes afin de les débarrasser des cellules et des débris cellulaires flottants.

Les surnageants ont alors été centrifugés à 20000g pendant 120 minutes à 4°C.

Le culot d'EMP a alors été lavé 2 fois avec du tampon phosphate salin (PBS) et re-suspendu dans du PBS. Des aliquotes de 10 µl de suspension d'EMP, dilué au 1/100, ont été marqués par de l'annexine V conjuguée à de l'isothiocyanate de fluorescéine (FITC) (Abcys, Paris, France). Les EMPs ont été dénombrées par cytométrie de flux comme préalablement décrit dans J. Thromb. Haemost. 2004 Oct; 2(10):1842-3 et demande de brevet français FR-A-2795820.

### 1-B : Immobilisation des EMP

Les EMP ont été immobilisées sur une surface en polycation selon le principe de l'adsorption physico-chimique.

Pour cela les parois et le fond des puits à fond rond de plaques 96 puits en PVC ont été activés avec 25µg/ml poly-L-lysine (Aldrich-Sigma). Différentes concentrations d'EMP dans du PBS ont alors été incubées pendant une nuit à 4°C dans les puits préalablement activés. Les puits sont alors lavés et les EMP immobilisées ont été utilisées dans le test de génération de plasmine.

### 1-C : Test de génération de plasmine

### 1-C-1 : protocole

Dans les puits de plaques 96 puits à fond rond en PVC, différentes concentrations d'EMP en suspension dans du PBS additionné de 0, 8% sérum albumine bovine (PBSA) ont été incubées avec 50 µl d'un mélange de plasminogène 1 µM et de (methylmalonyl)-hydroxypropylarginine-p-nitroanilide (CBS0065, Stago, Asnières, France), substrat chromogénique sélectif de la plasmine, à 0, 75 mM.

Un volume identique de surnageant du dernier lavage des EMP a été utilisé comme contrôle.

La microplaque est déposée dans le lecteur de microplaques et la cinétique de l'apparition de la plasmine a été suivie pendant 9 heures à l'aide d'un spectrophotomètre adapté à la lecture des plaques multi puits (MX5000, Dynex) à 37°C par mesure des modifications de l'absorbance à 405 nm produites par le relargage de la p-nitroaniline en fonction du temps.

### 1-C-2 : Résultats

Les résultats de ces mesures sont présentés dans le tableau suivant :

| **EMP/50µL** | **A405nm/min** |
|---|---|
| 10⁶ | 48, 7 |
| 10⁵ | 12, 9 |
| 5.10⁴ | 4, 7 |
| 10⁴ | 1, 6 |
| 10³ | 1, 1 |
| 0 | 0, 5 |
| 5.10⁴+EACA | 0, 6 |

EACA : acide ε-aminocaproique (ε-aminocaproic acid), inhibiteur de la liaison du plasminogène aux MPs.

### 1-D : Mesure de la constante de Michaelis

### 1-D-1 : mesure sur des EMP en suspension

### 1-D-1a : protocole

Dans les puits de plaques 96 puits à fond rond en PVC, 2.10⁵ EMP en suspension dans du PBS additionné de 0, 8% sérum albumine bovine (PBSA) ont été incubées avec différentes concentrations de plasminogène (0 to 5 µM) dans un volume final de 50 µl en présence de (methyl-malonyl)-hydroxypropylarginine-p-nitroanilide (CBS0065, Stago, Asnières, France), substrat chromogénique sélectif de la plasmine, à 0, 75 mM.

Un volume identique de surnageant du dernier lavage des EMP a été utilisé comme contrôle.

### 1-D-1b : Résultats

Les résultats de ces mesures sont présentés dans le tableau suivant :

| **Pg (µM)** | **A405nm/min** |
|---|---|
| 5 | 36, 8 |
| 2, 5 | 31, 8 |
| 1, 25 | 25, 9 |
| 0, 62 | 21, 8 |
| 0, 31 | 18, 2 |
| 0, 18 | 16, 4 |
| 0 | 0, 4 |

Par application de l'équation de Michaelis-Menten, ces résultats permettent de déterminer la constante de Michaelis de la génération spécifique de plasmine : Km = 0, 122 µM

### 1-D-2 : mesure sur des EMP immobilisées

### 1-D-2a : protocole

Les microparticules ont été immobilisées dans les puits comme indiqué ci-dessus

### (1-B Immobilisation de microparticules).

Le plasminogène et le substrat chromogène ont été ajoutés aux microparticules immobilisées selon le même protocole que pour les EMP en suspension.

La cinétique de formation de plasmine est détectée dans un lecteur de microplaques par mesure de l'absorbance à 405 nm.

Cette variante permet après détection de la cinétique d'activation de mesurer la plasmine liée aux microparticules immobilisées. A cet effet, les plaques sont lavées avec du PBSA et la plasmine fixée aux microparticules immobilisées a été détectée par addition de 50 µl/puits de 0, 325 mM de CBS0065 et mesure de modifications de l'absorbance à 405 nm.

### 1-D-2b : Résultats

| **EMP/50µL** | **A405nm/min** |
|---|---|
| 2.10⁵ | 4, 6 |
| 10⁵ | 2, 3 |
| 7.5.10⁴ | 1, 5 |
| 5.10⁴ | 0, 8 |
| 2.5.10⁴ | 0, 6 |

### 1-E : Conclusion

Ces résultats montrent que la formation de plasmine par les microparticules est fonction du nombre de microparticules ajoutées aux puits ou à concentration fixe de microparticules de la concentration de plasminogène ajoutée. Ces résultats montrent également que l'effet des microparticules est du à la présence d'un activateur du plasminogène présent sur les microparticules.

### Exemple 2 : Mise en évidence de l'activité plasmine portée par les microparticules in vivo :

### 2-A : Protocole

A partir d'un échantillon de sang total préalablement obtenu d'un individu présentant une pathologie autoimmune à risque thrombotique on isole les microparticules selon la méthode suivante :
+ (étape 1A) on centrifuge 2 ml dudit échantillon de sang à la vitesse de 1500g, pendant 10 minutes, à la température de 4°C;
+ (étape 1B) on centrifuge le surnageant obtenu à l'étape 1A à la vitesse de 17500g, pendant 2 minutes, à la température de 4°C ;
+ (étape 1 C) on centrifuge le surnageant obtenu à l'étape 1 B à la vitesse de 17500g, pendant 90 minutes, à la température de 4°C ;
+ (étape 1D) on reprend le culot obtenu à l'étape 1C dans 1000 µl de tampon phosphate salin (PBS) et on centrifuge le mélange à la vitesse de 17500g, pendant 90 minutes, à la température de 4°C ;
+ (étape 1 E) on reprend le culot obtenu à l'étape 1D dans 1000 µl de tampon phosphate salin (PBS) et on centrifuge le mélange à la vitesse de 17500g, pendant 90 minutes, à la température de 4°C;
+ (étape 1 F) on reprend le culot obtenu à l'étape 1E dans 50 µl de tampon phosphate salin (PBS) pour conservation et utilisation ultérieure.

Les microparticules ainsi obtenues à l'étape 1 F sont dénombrées par cytométrie de flux.

Dans une cupule à fond rond d'une plaque 96 puits (Vinyl alphanumeric U bottom plates, Ref. 2101, Thermo), dans un volume final de 50 µl, ajusté si nécessaire par du tampon phosphate salin (PBS) additionné de sérumalbumine bovine à la concentration finale de 2 mg/ml, à 200000 microparticules précédemment obtenues et préservées dans du PBS additionné de sérumalbumine bovine à la concentration finale de 2 mg/ml, on ajoute du plasminogène purifié (Américan Diagnostica, Hyphen) à une concentration finale de 0, 5 µM (ou 1µM) et du CBS0065 (STAGO) à la concentration finale de 0, 75 mM.

A l'issu de l'addition du plasminogène et du substrat chromogène aux microparticules dans un volume final de 50 µl dans la plaque de 96 puits, ladite plaque est placée directement dans le photomètre thermostaté à 37°C (MX5000, Dynex) afin de détecter la variation de l'absorbance à 405 nm en fonction du temps pendant 4 à 8 heures.

L'activité plasmine d'un échantillon témoin, provenant d'un sujet exempt de risque thrombotique est mesurée en parallèle dans les mêmes conditions. La quantité de plasmine produite par les microparticules est calculée par rapport à une courbe de référence faite avec des concentrations variables de plasmine (0 à 20 nM).

### 2-B : Résultats

| P | A | P | A | P | A | P | A | P | A | P | A |
|---|---|---|---|---|---|---|---|---|---|---|---|
| 1 | 1, 35 | 5 | 3, 5 | 9 | 0, 4 | 13 | 1, 5 | 17 | 3, 75 | 21 | 1, 5 |
| 2 | 1, 8 | 6 | 1, 0 | 10 | 0, 7 | 14 | 1, 05 | 18 | 7, 5 | 22 | 0, 9 |
| 3 | 0, 7 | 7 | 0, 6 | 11 | 1, 2 | 15 | 1, 8 | 19 | 6, 5 | | |
| 4 | 0, 5 | 8 | 2, 8 | 12 | 9, 05 | 16 | 2, 15 | 20 | 0, 85 | | |

| | | | | | | | | | | | |
|---|---|---|---|---|---|---|---|---|---|---|---|
| A : absorbance (405 nm/min) ; P = patient | | | | | | | | | | | |

### 2-C : Conclusion

Ces résultats montrent que les microparticules circulantes isolées à partir du plasma d'un sujet présentant une maladie autoimmune, génèrent de la plasmine comme les particules du prototype testé in vitro. Ces résultats montrent également que l'effet des microparticules produites in vivo est dépendant de la présence de plasminogène ajouté.

## Revendications

1. Méthode de mesure de l'activité plasmine des microparticules isolées, particulièrement les microparticules circulantes, d'un échantillon de sang, préalablement prélevé, dans lequel
- dans une première étape on isole lesdites microparticules présentes dans ledit échantillon, selon un procédé dans lequel dans
o une étape 1A on centrifuge un volume compris entre 500 µl et 5 ml, d'un échantillon de sang, préalablement prélevé, à une vitesse comprise entre 1000 g et 2000 g, pendant un temps compris entre 5 minutes et 20 minutes, à une température comprise entre 2 et 6°C ;
o dans une étape 1 B, on centrifuge le surnageant obtenu à l'étape 1A à une vitesse comprise entre 10000 g et 20000 g, pendant un temps compris entre 1 minute et 5 minutes, à une température comprise entre 2 et 6°C ;
o dans une étape 1 C on centrifuge le surnageant obtenu à l'étape 1 B à une vitesse comprise entre 15000 g et 25000 g, pendant un temps compris entre 45 et 120 minutes, à une température comprise entre 2 et 6°C ;
o dans une étape 1D on reprend le culot obtenu à l'étape 1C dans un volume compris entre 250 µl et 4 ml de tampon phosphate salin (PBS) et on centrifuge le mélange à une vitesse comprise entre 15000 g et 25000 g pendant un temps compris entre 45 et 120 minutes à une température comprise entre 2 et 6°C ;
o dans une étape 1E on reprend le culot obtenu à l'étape 1D dans un volume compris entre 250 µl et 4 ml de tampon phosphate salin (PBS) et on centrifuge le mélange à une vitesse comprise entre 15000 g et 25000 g pendant un temps compris entre 45 et 120 minutes à une température comprise entre 2 et 6°C ;
o dans une étape 1F on reprend le culot obtenu à l'étape 1E dans un volume compris entre 20 µl et 500 µl de tampon phosphate salin (PBS).
- dans une deuxième étape on mesure, par tout moyen approprié, la capacité desdites microparticules isolées à l'étape 1 à générer de la plasmine et
- dans une troisième étape on compare le résultat de la mesure obtenu à l'étape 2 au résultat d'une mesure identique réalisée dans les mêmes conditions sur un échantillon de sang identique témoin.

2. Méthode selon la revendication 1, **caractérisée en ce que** ledit échantillon de sang identique témoin est un échantillon de sang provenant d'au moins un individu considéré comme sain, ou est du sang, provenant du même individu mais obtenu dans un prélèvement antérieur à celui ayant donné l'échantillon testé, par exemple avant le début d'un traitement.

3. Méthode selon l'une quelconque des revendications 1 ou 2, **caractérisée en ce que** la première étape de ladite méthode (isolement des microparticules, particulièrement les microparticules circulantes, présentes dans l'échantillon), est réalisée selon un procédé dans lequel dans
+ à l'étape 1A le volume de l'échantillon de sang, centrifugé est préférentiellement compris entre 1 ml et 2 ml, à une vitesse préférentiellement comprise entre 1200 g et 1800g, pendant un temps compris préférentiellement entre 10 et 15 minutes, à une température comprise préférentiellement entre 3 et 5°C ;
+ à l'étape 1B, on centrifuge le plasma ou surnageant obtenu à l'étape 1A à une vitesse comprise préférentiellement entre 12000 g et 15000g, pendant un temps compris préférentiellement entre 2 et 3 minutes, à une température comprise préférentiellement entre 3 et 5°C ;
+ à l'étape 1C on centrifuge le surnageant obtenu à l'étape 1 B à une vitesse comprise préférentiellement entre 18000 g et 22000g, pendant un temps compris préférentiellement entre 60 et 100 minutes, à une température comprise préférentiellement entre 3 et 5°C ;
+ à l'étape 1D on reprend le culot obtenu à l'étape 1C dans un volume compris préférentiellement entre 1 et 2 ml, de tampon phosphate salin (PBS) et on centrifuge le mélange à une vitesse comprise préférentiellement entre 18000 g et 22000g, pendant un temps compris préférentiellement entre 60 et 100 minutes, à une température comprise préférentiellement entre 3 et 5°C ;
+ à une étape 1 E on reprend le culot obtenu à l'étape 1 D dans un volume compris préférentiellement entre 1 et 2 ml, de tampon phosphate salin (PBS) et on centrifuge le mélange à une vitesse comprise préférentiellement entre 18000 g et 22000g, pendant un temps compris préférentiellement entre 60 et 100 minutes, à une température comprise entre 2 et 6°C, préférentiellement entre 3 et 5°C ;
+ à une étape 1F on reprend le culot obtenu à l'étape 1 E dans un volume compris préférentiellement entre 50 et 100 µl, de tampon phosphate salin (PBS).

4. Méthode selon l'une quelconque des revendications 1 à 3, **caractérisée en ce que** la deuxième étape de ladite méthode (mesure de la capacité desdites microparticules à générer de la plasmine) est réalisée directement sur la quantité de microparticules obtenues à l'issu de la première étape.

5. Méthode selon l'une quelconque des revendications 1 à 3, **caractérisée en ce que** la deuxième étape de ladite méthode (mesure de la capacité desdites microparticules à générer de la plasmine) est réalisée sur une quantité déterminée de microparticules obtenues à l'issu de la première étape.

6. Méthode selon la revendication 5, **caractérisée en ce que**, ladite quantité est comprise entre 10000 et 1000000 microparticules, préférentiellement entre 100000 et 300000 microparticules.

7. Méthode selon l'une quelconque des revendications 1 à 3 ou 5 ou 6, **caractérisée en ce qu'**elle comporte une étape supplémentaire (étape 1 bis) de dénombrement desdites microparticules obtenues à l'issu de la première étape, ladite étape de dénombrement intervenant entre la première et la deuxième étape de la méthode.

8. Méthode selon la revendication 7, **caractérisée en ce que** le dénombrement desdites microparticules est réalisé par cytométrie de flux.

9. Méthode selon l'une quelconque des revendications 1 à 8, **caractérisée en ce que** la deuxième étape de ladite méthode, c'est-à-dire la mesure de la capacité desdites microparticules isolées à l'étape 1 à générer de la plasmine est déterminée soit par la mesure de la quantité de plasmine spontanément présente sur lesdites microparticules, soit par mesure de la quantité de plasmine susceptible d'être produite par ces microparticules.

10. Méthode selon la revendication 9, **caractérisée en ce que** la mesure de la quantité de plasmine spontanément présente sur lesdites microparticules, est réalisée par toute méthode connue comme par exemple une mesure immunologique à l'aide d'anticorps antiplasmin(ogèn)e ou encore par spectrophotométrie par lecture de l'absorbance de l'échantillon à 405 nm a l'aide de substrats chromogènes sélectifs de la plasmine.

11. Méthode selon la revendication 9, **caractérisée en ce que** la mesure de la quantité de plasmine susceptible d'être produite par lesdites microparticules est réalisée selon un procédé dans lequel
- dans une étape 2-1 on ajoute aux microparticules obtenues à l'étape 1 ou à l'étape 1 bis de ladite méthode, du plasminogène, avantageusement purifié, en une quantité finale comprise entre 0, 1 µM et 2, 0 µM, préférentiellement entre 0, 5 µM et 1 µM, et un substrat chromogénique sélectif de la plasmine, en une quantité finale comprise entre 0, 50 mM et 1, 0 mM, préférentiellement entre 0, 65 mM et 0, 85 mM
- dans une étape 2-2 on incube, le mélange obtenu à l'étape 2-1, par exemple dans une étuve sèche, à une température comprise entre 25°C et 45°C, préférentiellement 30°C et 40°C, pendant un temps compris entre 30 minutes et 90 minutes, préférentiellement entre 50 et 70 minutes et
- dans une étape 2-3 la quantité de plasmine susceptible d'être produite est détectée par photométrie par lecture de l'absorbance de l'échantillon à 405 nM.

12. Méthode selon la revendication 11, **caractérisée en ce que** de l'étape 2-1, le substrat sélectif de la plasmine est un substrat fluorescent comme par exemple le H-D-Val-Leu-Lys-7-amido-4-methylcoumarin (Bachem, Bubendorf, Switzerland) ou le D-AFK-ANSNH-iC4H9.2HB (Haematologic Technologies Inc, Vermont USA).

13. Méthode selon l'une quelconque des revendications 1 à 12, **caractérisée en ce que** la deuxième étape de ladite méthode, c'est-à-dire la mesure de la capacité desdites microparticules isolées à l'étape 1 à générer de la plasmine, est réalisée dans un volume final compris entre 25 µl et 150 µl, préférentiellement entre 50 µl et 100 µl.

14. Méthode selon l'une quelconque des revendications 1 à 13, **caractérisée en ce que** la deuxième étape de ladite méthode, c'est-à-dire la mesure de la capacité desdites microparticules isolées à l'étape 1 à générer de la plasmine, est réalisée dans un tampon phosphate salin (PBS) additionné de sérum albumine bovine (Bovine Serum Albumin : BSA) à une concentration comprise entre 1, 0 et 3, 0 mg/ml, préférentiellement entre 1, 5 et 2, 5 mg/ml.

15. Méthode selon l'une quelconque des revendications 1 à 14, **caractérisée en ce qu'**elle comprend en outre une étape 1ter d'isolement desdites microparticules en fonction de leur origine.

16. Méthode selon l'une quelconque des revendications 1 à 15, **caractérisée en ce que** lesdites microparticules isolées à l'étape 1 sont immobilisées au support sur lequel l'étape 2 est réalisée.

17. Méthode selon la revendication 16, **caractérisée en ce que** lesdites microparticules isolées à l'étape 1 sont immobilisées au support à l'aide d'un composé apte à immobiliser lesdites microparticules, ledit composé étant préalablement fixé à la surface dudit support.

18. Méthode selon la revendication 17, **caractérisée en ce que** ledit composé apte à immobiliser lesdites microparticules est choisi parmi l'annexine V, les anticorps spécifiques des complexes glycoprotéiques conformationnels actifs et/ou fonctionnels des membranes GPIIb/GPIIIa, les récepteurs adhésifs des monocytes ou des lymphocytes LFA-1, la thrombomoduline endothéliale, ou encore le CD 146 ou encore un polycation comme la poly-L-lysine.

19. Utilisation de microparticules circulantes présentes dans un échantillon de sang, dans une méthode de mesure de l'activité plasmine dudit échantillon de sang selon l'une quelconque des revendications 1 à 18.

20. Utilisation d'une méthode selon l'une quelconque des revendications 1 à 18, dans une méthode de diagnostic, chez un individu dont le sang est issu,
- du risque plus ou moins élevé à subir des accidents vasculaires causés par exemple par une instabilité accrue des plaques d'athérome, ou encore
- du risque plus ou moins élevé pour ledit individu atteint d'un cancer à subir une invasion métastasique, ou encore
- du risque plus ou moins élevé pour ledit individu à subir un accident vasculaire cérébral et ses conséquences hémorragiques, ou encore
- du risque pour ledit individu de subir une thrombose.

## Claims

1. Method for measuring the plasmin activity of isolated microparticles, in particular the circulating microparticles, in a sample of blood, taken previously, in which
- in a first step said microparticles present in said sample are isolated, following a procedure in which in
o a step 1A a volume comprised between 500 µl and 5 ml, of a blood sample, taken previously, is centrifuged at a speed comprised between 1,000 g and 2,000 g, for a time comprised between 5 minutes and 20 minutes at a temperature comprised between 2 and 6°C;
o in a step 1 B the supernatant obtained in step 1A is centrifuged at a speed comprised between 10,000 g and 20,000 g, for a time comprised between 1 minute and 5 minutes at a temperature comprised between 2 and 6°C;
o in a step 1C the supernatant obtained in step 1 B is centrifuged at a speed comprised between 15,000 g and 25,000 g, for a time comprised between 45 and 120 minutes at a temperature comprised between 2 and 6°C;
o in a step 1D the pellet obtained in step 1C is taken up in a volume comprised between 250 µl and 4 ml of phosphate buffered saline (PBS) and the mixture is centrifuged at a speed comprised between 15,000 g and 25,000 g, for a time comprised between 45 and 120 minutes at a temperature comprised between 2 and 6°C;
o in a step 1 E the pellet obtained in step 1D is taken up in a volume comprised between 250 µl and 4 ml of phosphate buffered saline (PBS) and the mixture is centrifuged at a speed comprised between 15,000 g and 25,000 g, for a time comprised between 45 and 120 minutes at a temperature comprised between 2 and 6°C;
o in a step 1 F the pellet obtained in step 1 E is taken up in a volume comprised between 20 µl and 500 µl of phosphate buffered saline (PBS).
- in a second step, the ability of said microparticles isolated in step 1 to generate plasmin is measured by any appropriate means and
- in a third step, the result of the measurement obtained in step 2 is compared with the result of an identical measurement carried out under the same conditions on a control sample of identical blood.

2. Method according to claim 1, **characterized in that** said control sample of identical blood is a blood sample originating from at least one individual considered as healthy, or is blood originating from the same individual but obtained in a sampling prior to that having produced the tested sample, for example before the start of a treatment.

3. Method according to any one of claims 1 or 2, **characterized in that** the first step of said method (isolation of the microparticles, in particular the circulating microparticles, present in the sample) is carried out according to a method in which in
+ step 1A the volume of the blood sample centrifuged is preferentially comprised between 1 ml and 2 ml, at a speed preferentially comprised between 1,200 g and 1,800g, for a time preferentially comprised between 10 and 15 minutes, at a temperature preferentially comprised between 3 and 5°C;
+ step 1 B the plasma or supernatant obtained in step 1A is centrifuged at a speed preferentially comprised between 12,000 g and 15,000g, for a time preferentially comprised between 2 and 3 minutes, at a temperature preferentially comprised between 3 and 5°C;
+ step 1C the supernatant obtained in step 1 B is centrifuged at a speed preferentially comprised between 18,000 g and 22,000g, for a time preferentially comprised between 60 and 100 minutes, at a temperature preferentially comprised between 3 and 5°C;
+ step 1D the pellet obtained in step 1C is taken up in a volume preferentially comprised between 1 and 2 ml of phosphate buffered saline (PBS) and the mixture is centrifuged at a speed preferentially comprised between 18,000 g and 22,000 g, for a time preferentially comprised between 60 and 100 minutes, at a temperature preferentially comprised between 3 and 5°C;
+ a step 1 E the pellet obtained in step 1D is taken up in a volume preferentially comprised between 1 and 2 ml of phosphate buffered saline (PBS) and the mixture is centrifuged at a speed preferentially comprised between 18,000 g and 22,000g, for a time preferentially comprised between 60 and 100 minutes, at a temperature comprised between 2 and 6°C, preferentially between 3 and 5°C;
+ a step 1 F the pellet obtained in step 1 E is taken up in a volume preferentially comprised between 50 and 100 µl, of phosphate buffered saline (PBS).

4. Method according to any one of claims 1 to 3, **characterized in that** the second step of said method (measurement of the ability of said microparticles to generate plasmin) is carried out directly on the quantity of microparticles obtained on completion of the first step.

5. Method according to any one of claims 1 to 3, **characterized in that** the second step of said method (measurement of the ability of said microparticles to generate plasmin) is carried out on a determined quantity of microparticles obtained on completion of the first step.

6. Method according to claim 5, **characterized in that** said quantity is comprised between 10,000 and 1,000,000 microparticles, preferentially between 100,000 and 300,000 microparticles.

7. Method according to any one of claims 1 to 3 or 5 or 6, **characterized in that** it comprises an additional step (step 1a) of counting said microparticles obtained on completion of the first step, said counting step occurring between the first and the second step of the method.

8. Method according to claim 7, **characterized in that** the counting of said microparticles is carried out by flow cytometry.

9. Method according to any one of claims 1 to 8, **characterized in that** the second step of said method, i.e. the measurement of the ability of said microparticles isolated in step 1 to generate plasmin is determined either by measurement of the quantity of plasmin spontaneously present on said microparticles, or by measurement of the quantity of plasmin capable of being produced by these microparticles.

10. Method according to claim 9, **characterized in that** the measurement of the quantity of plasmin spontaneously present on said microparticles is carried out by any known method such as for example an immunological measurement using antiplasmin(ogen) antibodies or also by spectrophotometry by absorbance reading of the sample at 405 nm using chromogenic substrates selective for plasmin.

11. Method according to claim 9, **characterized in that** the measurement of the quantity of plasmin capable of being produced by said microparticles is carried out according to a procedure in which
- in a step 2-1, to the microparticles obtained in step 1 or in step 1 a of said method are added plasminogen, advantageously purified, in a final quantity comprised between 0.1 µM and 2.0 µM, preferentially between 0.5 µM and 1 µM, and a chromogenic substrate selective for plasmin, in a final quantity comprised between 0.50 mM and 1.0 mM, preferentially between 0.65 mM and 0.85 mM;
- in a step 2-2 the mixture obtained in step 2-1, for example in a drying oven, is incubated at a temperature comprised between 25°C and 45°C, preferentially 30°C and 40°C, for a time comprised between 30 minutes and 90 minutes, preferentially between 50 and 70 minutes and
- in a step 2-3 the quantity of plasmin capable of being produced is detected by photometry by absorbance reading of the sample at 405 nM.

12. Method according to claim 11, **characterized in that** in step 2-1, the plasmin-selective substrate is a fluorescent substrate such as for example H-D-Val-Leu-Lys-7-amido-4-methylcoumarin (Bachem, Bubendorf, Switzerland) or D-AFK-ANSNH-iC4H9.2HB (Haematologic Technologies Inc, Vermont USA).

13. Method according to any one of claims 1 to 12, **characterized in that** the second step of said method, i.e. the measurement of the ability of said microparticles isolated in step 1 to generate plasmin, is carried out in a final volume comprised between 25 µl and 150 µl, preferentially between 50 µl and 100 µl.

14. Method according to any one of claims 1 to 13, **characterized in that** the second step of said method, i.e. the measurement of the ability of said microparticles isolated in step 1 to generate plasmin, is carried out in a phosphate-buffered saline (PBS) with bovine serum albumin (BSA) added, at a concentration comprised between 1.0 and 3.0 mg/ml, preferentially between 1.5 and 2.5 mg/ml.

15. Method according to any one of claims 1 to 14, **characterized in that** it also comprises a step 1 b of isolation of said microparticles as a function of their origin.

16. Method according to any one of claims 1 to 15, **characterized in that** said microparticles isolated in step 1 are immobilized on the support on which step 2 is carried out.

17. Method according to claim 16, **characterized in that** said microparticles isolated in step 1 are immobilized on the support using a compound capable of immobilizing said microparticles, said compound being previously fixed to the surface of said support.

18. Method according to claim 17, **characterized in that** said compound capable of immobilizing said microparticles is chosen from annexin V, the antibodies specific to the active and/or functional conformational glycoprotein complexes of the GPIIb/GPIIIa membranes, the adhesive receptors of the monocytes or of the LFA-1 lymphocytes, endothelial thrombomodulin, or also CD 146 or also a polycation such as poly-L-lysine.

19. Use of circulating microparticles present in a blood sample, in a method for measuring the plasmin activity of said blood sample according to any one of claims 1 to 18.

20. Use of a method according to any one of claims 1 to 18, in a diagnosis method, in an individual from whom the blood originates,
- of the greater or lesser risk of suffering vascular accidents caused for example by increased instability of the atheromatous plaques, or also
- of the greater or lesser risk of said individual with cancer suffering a metastatic invasion, or also
- of the greater or lesser risk of said individual suffering a cerebral vascular accident and its haemorrhagic consequences, or also
- of the risk of said individual suffering a thrombosis.

## Patentansprüche

1. Verfahren zum Messen der Plasminaktivität von isolierten Mikropartikeln, insbesondere von zirkulierenden Mikropartikeln, an einer zuvor genommenen Blutprobe, bei dem:
- in einem ersten Schritt die genannten in der genannten Probe vorhandenen Mikropartikel in einem Prozess isoliert werden, das die folgenden Schritte beinhaltet:
o einen Schritt 1A des Zentrifugierens eines Volumens zwischen 500 µl und 5 ml einer zuvor genommenen Blutprobe mit einer Geschwindigkeit zwischen 1000 g und 2000 g für eine Zeit zwischen 5 Minuten und 20 Minuten bei einer Temperatur zwischen 2 und 6°C;
o einen Schritt 1 B des Zentrifugierens des in Schritt 1A erhaltenen Überstands mit einer Geschwindigkeit zwischen 10.000 g und 20.000 g für eine Zeit zwischen 1 Minute und 5 Minuten bei einer Temperatur zwischen 2 und 6°C;
o einen Schritt 1C des Zentrifugierens des in Schritt 1 B erhaltenen Überstands mit einer Geschwindigkeit zwischen 15.000 g und 25.000 g für eine Zeit zwischen 45 und 120 Minuten bei einer Temperatur zwischen 2 und 6°C;
o einen Schritt 1D des Aufnehmens des in Schritt 1C erhaltenen Rückstands in ein Volumen zwischen 250 µl und 4 ml Phosphatsalinepuffer (PBS) und des Zentrifugierens des Gemischs mit einer Geschwindigkeit zwischen 15.000 g und 25.000 g für eine Zeit zwischen 45 und 120 Minuten bei einer Temperatur zwischen 2 und 6°C;
o einen Schritt 1 E des Aufnehmens des in Schritt 1D erhaltenen Rückstands in ein Volumen zwischen 250 µl und 4 ml Phosphatsalinepuffer (PBS) und des Zentrifugierens des Gemischs mit einer Geschwindigkeit zwischen 15.000 g und 25.000 g für eine Zeit zwischen 45 und 120 Minuten bei einer Temperatur zwischen 2 und 6°C;
o einen Schritt 1 F des Aufnehmens des in Schritt 1 E erhaltenen Rückstands in ein Volumen zwischen 20 µl und 500 µl Phosphatsalinepuffer (PBS);
- in einem zweiten Schritt mit einem geeigneten Mittel die Kapazität der genannten in Schritt 1 isolierten Mikropartikel zum Erzeugen von Plasmin gemessen wird, und
- in einem dritten Schritt das Ergebnis der Messung aus Schritt 2 mit dem Ergebnis einer identischen Messung verglichen wird, die unter denselben Bedingungen an einer identischen Referenzblutprobe durchgeführt wurde.

2. Verfahren nach Anspruch 1, **dadurch gekennzeichnet, dass** die genannte identische Referenzblutprobe eine Blutprobe ist, die von wenigstens einer als gesund angesehenen Person stammt, oder eine Blutprobe, die von derselben Person, aber von einer früheren Entnahme als die getestete Probe stammt, z.B. vor Beginn einer Behandlung.

3. Verfahren nach Anspruch 1 oder 2, **dadurch gekennzeichnet, dass** der erste Schritt des genannten Verfahrens (Isolieren der Mikropartikel, insbesondere der zirkulierenden Mikropartikel in der Probe) mit einem Prozess durchgeführt wird, bei dem:
+ in Schritt 1A das Volumen der zentrifugierten Blutprobe vorzugsweise zwischen 1 ml und 2 ml liegt, eine Geschwindigkeit vorzugsweise zwischen 1200 g und 1800 g liegt, eine Zeit vorzugsweise zwischen 10 und 15 Minuten liegt und eine Temperatur vorzugsweise zwischen 3 und 5°C liegt;
+ in Schritt 1B das/der in Schritt 1A erhaltene Plasma oder Überstand mit einer Geschwindigkeit vorzugsweise zwischen 12.000 g und 15.000 g für eine Zeit vorzugsweise zwischen 2 und 3 Minuten bei einer Temperatur vorzugsweise zwischen 3 und 5°C zentrifugiert wird;
+ in Schritt 1C der in Schritt 1 B erhaltene Überstand mit einer Geschwindigkeit vorzugsweise zwischen 18.000 g und 22.000 g für eine Zeit vorzugsweise zwischen 60 und 100 Minuten bei einer Temperatur vorzugsweise zwischen 3 und 5°C zentrifugiert wird;
+ in Schritt 1D der in Schritt 1C erhaltene Rückstand in einem Volumen vorzugsweise zwischen 1 und 2 ml Phosphatsalinepuffer (PBS) aufgenommen wird und das Gemisch mit einer Geschwindigkeit vorzugsweise zwischen 18.000 g und 22.000 g für eine Zeit vorzugsweise zwischen 60 und 100 Minuten bei einer Temperatur vorzugsweise zwischen 3 und 5°C zentrifugiert wird;
+ in einem Schritt 1 E der in Schritt 1D erhaltene Rückstand in einem Volumen vorzugsweise zwischen 1 und 2 ml Phosphatsalinepuffer (PBS) aufgenommen wird und das Gemisch bei einer Geschwindigkeit vorzugsweise zwischen 18.000 g und 22.000 g für eine Zeit vorzugsweise zwischen 60 und 100 Minuten bei einer Temperatur zwischen 2 und 6°C, vorzugsweise zwischen 3 und 5°C zentrifugiert wird;
+ in einem Schritt 1 F der in Schritt 1 E erhaltene Rückstand in einem Volumen vorzugsweise zwischen 50 und 100 µl Phosphatsalinepuffer (PBS) aufgenommen wird.

4. Verfahren nach einem der Ansprüche 1 bis 3, **dadurch gekennzeichnet, dass** der zweite Schritt des genannten Verfahrens (Messen der Kapazität der genannten Mikropartikel zum Erzeugen von Plasmin) direkt an der am Ausgang des ersten Schrittes erhaltenen Mikropartikelmenge durchgeführt wird.

5. Verfahren nach einem der Ansprüche 1 bis 3, **dadurch gekennzeichnet, dass** der zweite Schritt des genannten Verfahrens (Messen der Kapazität der genannten Mikropartikel zum Erzeugen von Plasmin) an einer bestimmten Menge von am Ausgang des ersten Schrittes erhaltenen Mikropartikeln durchgeführt wird.

6. Verfahren nach Anspruch 5, **dadurch gekennzeichnet, dass** die genannte Menge zwischen 10.000 und 1.000.000 Mikropartikeln, vorzugsweise zwischen 100.000 und 300.000 Mikropartikeln beträgt.

7. Verfahren nach einem der Ansprüche 1 bis 3 oder 5 oder 6, **dadurch gekennzeichnet, dass** es einen Zusatzschritt (Schritt 1(a)) des Zählens der genannten am Ausgang des ersten Schritts erhaltenen Mikropartikel beinhaltet, wobei der genannte Zählschritt zwischen dem ersten und dem zweiten Schritt des Verfahrens durchgeführt wird.

8. Verfahren nach Anspruch 7, **dadurch gekennzeichnet, dass** das Zählen der genannten Mikropartikel durch Flusszytometrie durchgeführt wird.

9. Verfahren nach einem der Ansprüche 1 bis 8, **dadurch gekennzeichnet, dass** der zweite Schritt des genannten Verfahrens, d.h. das Messen der Kapazität der genannten in Schritt 1 isolierten Mikropartikel zum Erzeugen von Plasmin, entweder durch spontanes Messen der Menge an an den genannten Mikropartikeln vorhandenem Plasmin oder durch Messen der von diesen Mikropartikeln erzeugbaren Plasminmenge bestimmt wird.

10. Verfahren nach Anspruch 9, **dadurch gekennzeichnet, dass** das spontane Messen der an den genannten Mikropartikeln vorhandenen Plasminmenge mit einem bekannten Verfahren wie beispielsweise durch eine immunologische Messung mit Hilfe von Antiplasmin(ogen)-Antikörpern oder auch durch Spektrofotometrie durch Lesen der Absorbanz der Probe bei 405 nm mit Hilfe von selektiven chromogenen Substraten des Plasmins erfolgt.

11. Verfahren nach Anspruch 9, **dadurch gekennzeichnet, dass** das Messen der Menge an von den genannten Mikropartikeln erzeugbarem Plasmin mit einem Prozess erfolgt, das die folgenden Schritte beinhaltet:
- einen Schritt 2-1 des Zugebens zu den in Schritt 1 oder in Schritt 1 (a) des genannten Verfahrens erhaltenen Mikropartikeln von Plasminogen, vorzugsweise gereinigt, in einer Endmenge zwischen 0,1 µM und 2,0 µM, vorzugsweise zwischen 0,5 µM und 1 µM, und eines selektiven chromogenen Substrats des Plasmins in einer Endmenge zwischen 0,50 mM und 1,0 mM, vorzugsweise zwischen 0,65 mM und 0,85 mM;
- einen Schritt 2-2 des Inkubierens des in Schritt 2-1 erhaltenen Gemischs, z.B. in einem Trockenofen, bei einer Temperatur zwischen 25°C und 45°C, vorzugsweise 30°C und 40°C, für eine Zeit zwischen 30 Minuten und 90 Minuten, vorzugsweise zwischen 50 und 70 Minuten, und
- einen Schritt 2-3 des Erkennens der Menge an erzeugbarem Plasmin durch Fotometrie durch Lesen der Absorbanz der Probe bei 405 nM.

12. Verfahren nach Anspruch 11, **dadurch gekennzeichnet, dass** in Schritt 2-1 das selektive Substrat des Plasmins ein fluoreszentes Substrat wie z.B. H-D-Val-Leu-Lys-7-amido-4-methylcoumarin (Bachem, Bubendorf, Schweiz) oder D-AFK-ANSNH-iC4H9.2HB (Haematologic Technologies Inc, Vermont USA) ist.

13. Verfahren nach einem der Ansprüche 1 bis 12, **dadurch gekennzeichnet, dass** der zweite Schritt des genannten Verfahrens, d.h. das Messen der Kapazität der genannten in Schritt 1 isolierten Mikropartikel zum Erzeugen des Plasmins, in einem Endvolumen zwischen 25 µl und 150 µl, vorzugsweise zwischen 50 µl und 100 µl durchgeführt wird.

14. Verfahren nach einem der Ansprüche 1 bis 13, **dadurch gekennzeichnet, dass** der zweite Schritt des genannten Verfahrens, d.h. das Messen der Kapazität der genannten in Schritt 1 isolierten Mikropartikel zum Erzeugen des Plasmins, in einem Phosphatsalinepuffer (PBS), ergänzt mit bovinem Serumalbumin (BSA), bei einer Konzentration zwischen 1,0 und 3,0 mg/ml, vorzugsweise zwischen 1,5 und 2,5 mg/ml erfolgt.

15. Verfahren nach einem der Ansprüche 1 bis 14, **dadurch gekennzeichnet, dass** es darüber hinaus einen Schritt 1(b) des Isolierens der genannten Mikropartikel in Abhängigkeit von ihrem Ursprung beinhaltet.

16. Verfahren nach einem der Ansprüche 1 bis 15, **dadurch gekennzeichnet, dass** die genannten in Schritt 1 isolierten Mikropartikel auf dem Träger immobilisiert werden, auf dem Schritt 2 durchgeführt wird.

17. Verfahren nach Anspruch 16, **dadurch gekennzeichnet, dass** die genannten in Schritt 1 isolierten Mikropartikel auf dem Träger mittels einer Verbindung immobilisiert werden, die die genannten Mikropartikel immobilisieren kann, wobei die genannte Verbindung zuvor auf der Oberfläche des genannten Trägers fixiert wurde.

18. Verfahren nach Anspruch 17, **dadurch gekennzeichnet, dass** die genannte Verbindung, die die genannten Mikropartikel immobilisieren kann, ausgewählt ist aus Annexin V, den spezifischen Antikörpern der konformativen aktiven und/oder funktionellen Glykoproteinkomplexe der GPIIb/GPIIIa-Membrane, den haftenden Rezeptoren von LFA-1 Monocyten oder Lymphocyten, endothelialem Thrombomodulin oder auch CD 146 oder einem Polykation wie Poly-L-lysin.

19. Verwendung von in einer Blutprobe vorhandenen zirkulierenden Mikropartikeln in einem Verfahren zum Messen der Plasminaktivität der genannten Blutprobe nach einem der Ansprüche 1 bis 18.

20. Anwendung eines Verfahrens nach einem der Ansprüche 1 bis 18 in einem Verfahren zum Diagnostizieren bei einer Person, von der das Blut stammt,
- der Höhe des Risikos des Auftretens von Gefäßstörungen, die beispielsweise durch eine erhöhte Instabilität von atheromatösen Plaques verursacht werden, oder auch
- der Höhe des Risikos einer invasiven Metastasierung bei der genannten Person, wenn sie an Krebs erkrankt ist, oder auch
- der Höhe des Risikos des Auftretens einer Hirngefäßstörung und deren hämorrhagischen Konsequenzen für die genannte Person, oder auch
- des Thromboserisikos für die genannte Person.
